# EUROPEAN PATENT APPLICATION

(11) **EP 4 806 982 A1**
(43) Date of publication of application: **16.09.2026**
(21) Application number: 26164037.9
(22) Date of filing: 11.03.2026
(51) Int. Cl.: C12M 1/32, C12M 1/26

(54) **CELL HOUSING DEVICE**

(30) Priority: 12.03.2025 JP 2025039792
(71) Applicant: Yokogawa Electric Corporation, Musashino-shi, Tokyo 180-8750 (JP)
(72) Inventor: KITA, Mizuki, Musashino-shi, Tokyo, 180-8750 (JP); TANO, Shintaro, Musashino-shi, Tokyo, 180-8750 (JP); KANEKI, Shinsuke, Musashino-shi, Tokyo, 180-8750 (JP)
(74) Representative: Stöckeler, Ferdinand

(57) **Abstract**

A cell housing device that enables manipulation of the posture of a target cell accommodated in a well is provided. A cell housing device includes a well and a posture manipulation device. The well has inner wall surfaces that accommodate a target cell immersed in a liquid. The target cell is a cell or a cell aggregate. The posture manipulation device has a retention channel. The retention channel drains a fluid from the well via the inner wall surface of the well, to bring the target cell into a retained state in which the target cell is rotatably retained at a tip end of the retention channel. The posture manipulation device manipulates the posture of the target cell by rotating the target cell in the retained state.

## Description

### TECHNICAL FIELD

The present disclosure relates to a cell housing device.

### BACKGROUND

In the fields of regenerative medicine, drug development, and the like, cell housing devices with wells are used (see Patent Literatures (PTLs) 1 and 2). The wells are capable of accommodating target cells, such as cells or cell aggregates (spheroids; see PTLs 3 and 4), immersed in liquids.

### CITATION LIST

### Patent Literatures

PTL 1: JP 2022-105173 A
PTL 2: JP 2015-192644 A
PTL 3: JP 2016-105700 A
PTL 4: JP 2021-148618 A

### SUMMARY

It is convenient for users to manipulate the posture of target cells with cell housing devices themselves when substances are extracted from or injected into target cells accommodated in wells for the purpose of cell culture, manipulation, observation, or the like.

Therefore, the present disclosure aims to provide a cell housing device that enables manipulation of the posture of a target cell accommodated in a well.

One aspect of the present disclosure is as follows.
[1] A cell housing device including:
   a well; and
   a posture manipulation device,
   wherein
   the well has an inner wall surface configured to accommodate a target cell immersed in a liquid,
   the target cell is a cell or a cell aggregate,
   the posture manipulation device has a retention channel,
   the retention channel is configured to drain a fluid from the well via the inner wall surface of the well, to bring the target cell into a retained state in which the target cell is rotatably retained at a tip end of the retention channel, and
   the posture manipulation device is configured to manipulate a posture of the target cell by rotating the target cell in the retained state.
[2] The cell housing device according to [1], wherein
   the posture manipulation device has a manipulation channel, and
   the manipulation channel is configured to allow a fluid to flow via the inner wall surface of the well, to cause the target cell in the retained state to rotate in order to manipulate the posture of the target cell.
[3] The cell housing device according to [2], wherein the retention channel and the manipulation channel extend in parallel with each other.
[4] The cell housing device according to [2] or [3], wherein the retention channel and the manipulation channel extend at an angle to each other.
[5] The cell housing device according to [4], wherein a tip end of the manipulation channel merges with the tip end of the retention channel.
[6] The cell housing device according to any one of [2] to [4], wherein the retention channel and the manipulation channel are provided separately.
[7] The cell housing device according to any one of [2] to [6], wherein each of the retention channel and the manipulation channel is configured to allow a fluid to flow via a bottom wall surface of the well.
[8] The cell housing device according to any one of [2] to [7], wherein
   the manipulation channel corresponds to a first manipulation channel used for first posture manipulation in which the posture of the target cell is manipulated by rotating the target cell about a first axis,
   the posture manipulation device has a second manipulation channel, and
   the second manipulation channel configured to allow a fluid to flow via the inner wall surface of the well is used for second posture manipulation in which the posture of the target cell is manipulated by rotating the target cell in the retained state about a second axis different from the first axis.
[9] The cell housing device according to [1], wherein
   the retention channel extends into the well via a peripheral wall surface of the well, and
   the retention channel is used for intermittent retention manipulation in which the target cell is intermittently retained at the tip end of the retention channel using a fluid draining from the well, and the intermittent retention manipulation causes the target cell to rotate by the action of gravity or buoyancy to manipulate the posture of the target cell.
[10] The cell housing device according to [9], wherein
   the retention channel corresponds to a first retention channel used for first posture manipulation in which the posture of the target cell is manipulated by rotating the target cell about a first axis,
   the posture manipulation device has a second retention channel,
   the second retention channel extends into the well via the peripheral wall surface of the well, and
   the second retention channel is used for intermittent retention manipulation in which the target cell is intermittently retained at a tip end of the second retention channel using a fluid draining from the well, and the intermittent retention manipulation causes second posture manipulation to be performed in which the posture of the target cell is manipulated by rotating the target cell about a second axis different from the first axis by the action of gravity or buoyancy.

According to the present disclosure, it is possible to provide a cell housing device that enables manipulation of the posture of a target cell accommodated in a well.

### BRIEF DESCRIPTION OF THE DRAWINGS

In the accompanying drawings:
FIG. 1 is a cross-sectional view illustrating a cell housing device according to a first embodiment of the present disclosure;
FIG. 2 is a top view of the cell housing device illustrated in FIG. 1;
FIG. 3 is a cross-sectional view illustrating a cell housing device according to a second embodiment;
FIG. 4 is a top view of the cell housing device illustrated in FIG. 3;
FIG. 5 is a cross-sectional view illustrating a cell housing device according to a third embodiment;
FIG. 6 is a cross-sectional view illustrating a state of starting posture manipulation from the state illustrated in FIG. 5;
FIG. 7 is a cross-sectional view illustrating a state of completing the posture manipulation from the state illustrated in FIG. 6; and
FIG. 8 is a top view of the cell housing device illustrated in FIG. 5.

### DETAILED DESCRIPTION

Embodiments of the present disclosure will be exemplarily described in detail below, with reference to the drawings.

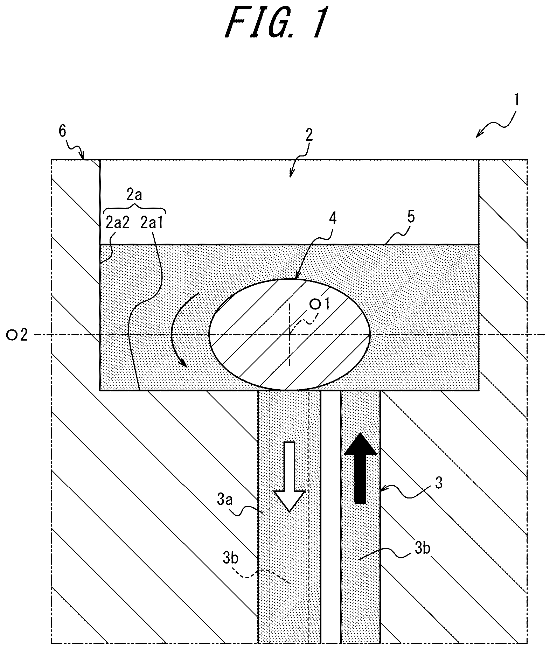

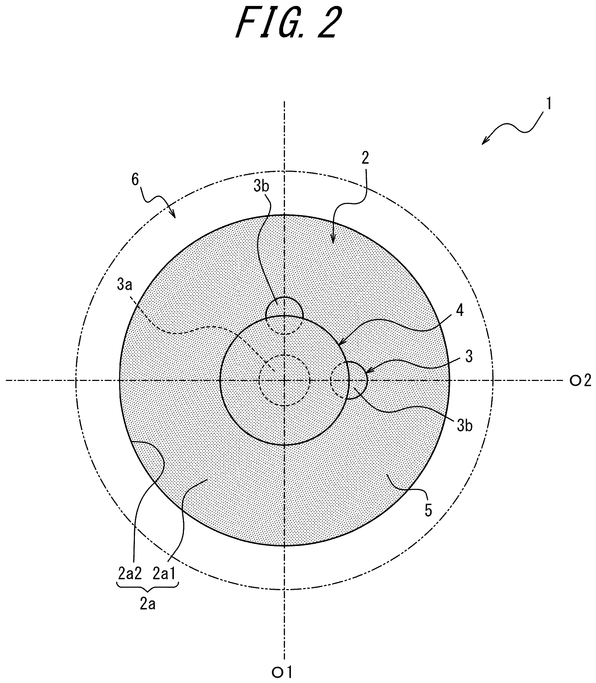

As illustrated in FIGS. 1 and 2, in a first embodiment of the present disclosure, a cell housing device 1 includes a well 2 and a posture manipulation device 3. The well 2 has inner wall surfaces 2a that accommodate a target cell 4 immersed in a liquid 5. The target cell 4 is a cell or a cell aggregate. The posture manipulation device 3 has a retention channel 3a. The retention channel 3a drains a fluid through an inner wall surface 2a of the well 2, as indicated by the white arrow in FIG. 1, to bring the target cell 4 into a retained state in which the target cell 4 is rotatably retained at a tip end of the retention channel 3a. The posture manipulation device 3 manipulates the posture of the target cell 4 by rotating the target cell 4 in the retained state. By draining the fluid from the well 2, the retention channel 3a causes a suction force generated at the tip end of the retention channel 3a to act on the target cell 4, so that the target cell 4 is brought into the retained state.

The cell is, for example, a human oocyte. The diameter of oocytes is usually of the order of 120 µm. The diameter of cell aggregates (spheroids) is usually of the order of 100 µm to 500 µm.

The posture manipulation device 3 has manipulation channels 3b. The manipulation channels 3b allow fluids to flow via the inner wall surface 2a of the well 2, to cause the target cell 4 in the retained state to rotate in order to manipulate the posture of the target cell 4. One of the manipulation channels 3b corresponds to a first manipulation channel 3b used for first posture manipulation in which the posture of the target cell 4 is manipulated by rotating the target cell 4 about a first axis O1. The other manipulation channel 3b corresponds to a second manipulation channel 3b. By allowing the fluid to flow via the inner wall surface 2a of the well 2, the second manipulation channel 3b is used for second posture manipulation in which the posture of the target cell 4 is manipulated by rotating the target cell 4 in the retained state about a second axis O2 different from the first axis O1.

In the first posture manipulation, the first manipulation channel 3b introduces the fluid into the well 2 through the inner wall surface 2a of the well 2, as indicated by the bold arrow in FIG. 1. In this case, by introducing the fluid into the well 2, the first manipulation channel 3b causes a pressure force generated at a tip end of the first manipulation channel 3b to act on the target cell 4 at a position offset from the center of gravity, so that the target cell 4 rotates about the first axis O1.

In the first posture manipulation, the first manipulation channel 3b may drain the fluid from the well 2 through the inner wall surface 2a of the well 2. In this case, by draining the fluid from the well 2, the first manipulation channel 3b causes a suction force generated at the tip end of the first manipulation channel 3b to act on the target cell 4 at a position offset from the center of gravity, so that the target cell 4 rotates about the first axis O1.

In the second posture manipulation, the second manipulation channel 3b introduces the fluid into the well 2 through the inner wall surface 2a of the well 2. In this case, by introducing the fluid into the well 2, the second manipulation channel 3b causes a pressure force generated at a tip end of the second manipulation channel 3b to act on the target cell 4 at a position offset from the center of gravity, so that the target cell 4 rotates about the second axis O2.

In the second posture manipulation, the second manipulation channel 3b may drain the fluid from the well 2 through the inner wall surface 2a of the well 2. In this case, by draining the fluid from the well 2, the second manipulation channel 3b causes a suction force generated at the tip end of the second manipulation channel 3b to act on the target cell 4 at a position offset from the center of gravity, so that the target cell 4 rotates about the second axis O2.

The retention channel 3a, the first manipulation channel 3b, and the second manipulation channel 3b extend in parallel with each other. The retention channel 3a, the first manipulation channel 3b, and the second manipulation channel 3b are provided separately.

The inner wall surfaces 2a of the well 2 have a bottom wall surface 2a1 and a peripheral wall surface 2a2 that extends upward from the outer edge of the bottom wall surface 2a1. The top of the well 2 is open, and the inner wall surfaces 2a of the well 2 are concave in shape. The peripheral wall surface 2a2 is circular in top view. The peripheral wall surface 2a2 is not limited to circular in top view, but may be, for example, rectangular, hexagonal, or the like. The bottom wall surface 2a1 is a flat surface. The bottom wall surface 2a1 is not limited to a flat surface, but may be, for example, a concave curved surface or the like.

Each of the retention channel 3a, the first manipulation channel 3b, and the second manipulation channel 3b allows the fluid to flow via the bottom wall surface 2a1 of the well 2. Each of the retention channel 3a, the first manipulation channel 3b, and the second manipulation channel 3b extends perpendicularly to the bottom wall surface 2a1 of the well 2. Each of the retention channel 3a, the first manipulation channel 3b, and the second manipulation channel 3b extends straight downward from the bottom wall surface 2a1 of the well 2.

Each of the retention channel 3a, the first manipulation channel 3b, and the second manipulation channel 3b may be configured to extend not perpendicularly but at an angle to the bottom wall surface 2a1 of the well 2. Each of the retention channel 3a, the first manipulation channel 3b, and the second manipulation channel 3b may be configured to extend curvedly downward from the bottom wall surface 2a1 of the well 2.

Each of the retention channel 3a, the first manipulation channel 3b, and the second manipulation channel 3b may be configured to allow the fluid to flow not via the bottom wall surface 2a1 of the well 2 but via the peripheral wall surface 2a2 of the well 2. One or two of the retention channel 3a, the first manipulation channel 3b, and the second manipulation channel 3b may be configured to allow the fluid to flow via the bottom wall surface 2a1 of the well 2, while the others allow the fluid to flow via the peripheral wall surface 2a2 of the well 2.

The well 2 is provided in a well container 6. The well container 6 can have a plurality of wells 2 arranged in any layout in a horizontal direction. Each of the retention channel 3a, the first manipulation channel 3b, and the second manipulation channel 3b is formed in the well container 6 itself. Each of the retention channel 3a, the first manipulation channel 3b, and the second manipulation channel 3b may be formed by a tube connected to the well container 6.

The fluid flowing through each of the retention channel 3a, the first manipulation channel 3b, and the second manipulation channel 3b is the liquid 5 in which the target cell 4 is immersed within the well 2. In the first posture manipulation, the first manipulation channel 3b may introduce, into the well 2, a different liquid 5 or gas from the liquid 5 in which the target cell 4 is immersed within the well 2. In the second posture manipulation, the second manipulation channel 3b may introduce, into the well 2, a different liquid 5 or gas from the liquid 5 in which the target cell 4 is immersed within the well 2.

The fluids flowing through the retention channel 3a, the first manipulation channel 3b, and the second manipulation channel 3b can be controlled using fluid delivery devices such as pumps. Operations of the fluid delivery devices can be controlled by a control device (not illustrated) . The control device can be configured by a computer with a processor and a memory. The control device controls fluid delivery operations, for example, based on input from an operator via an input terminal.

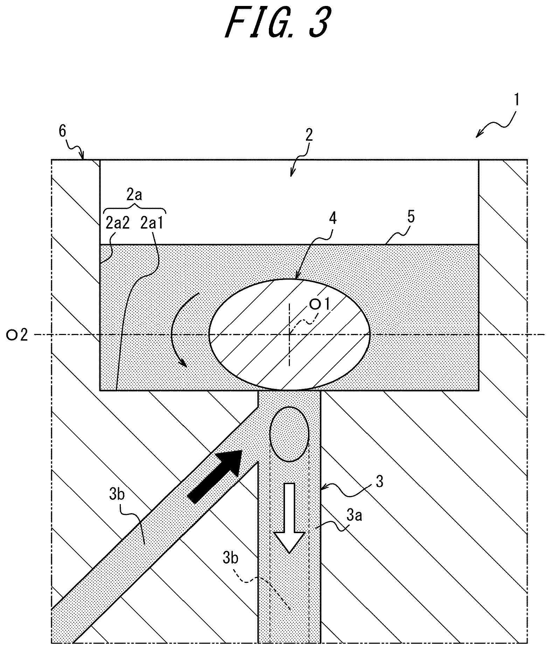

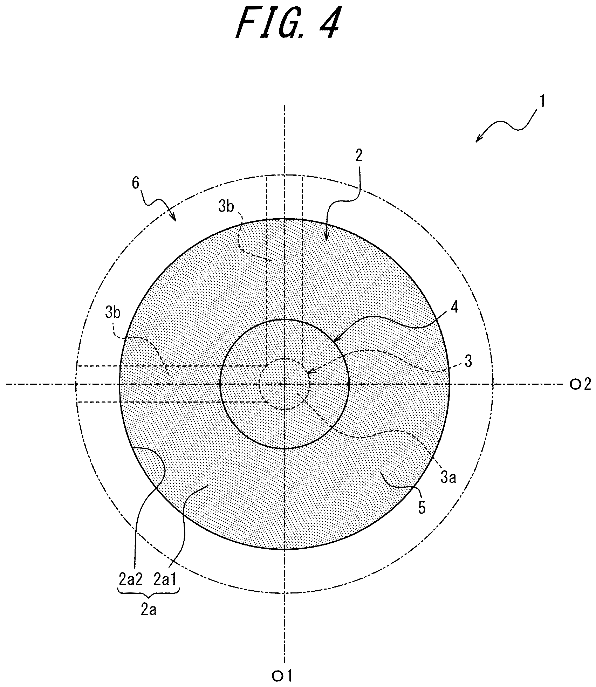

As in a second embodiment illustrated in FIGS. 3 and 4, the retention channel 3a, the first manipulation channel 3b, and the second manipulation channel 3b may be configured to extend at an angle to each other. In this case, as in the second embodiment, the tip ends of the first manipulation channel 3b and the second manipulation channel 3b may each be merged with the tip end of the retention channel 3a.

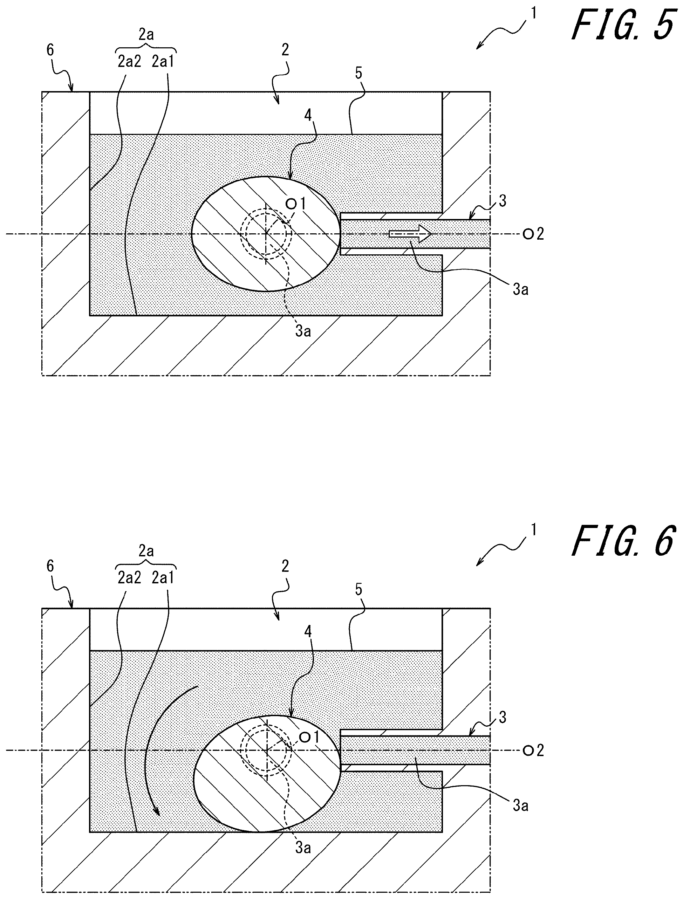

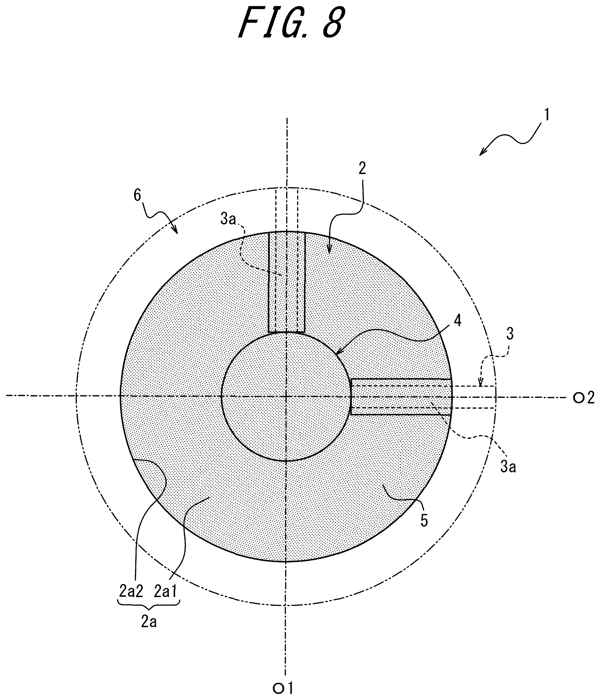

The posture manipulation device 3 may be configured to manipulate the posture of the target cell 4 by rotating the target cell 4 in the retained state, without using the manipulation channels 3b. For example, as in a third embodiment illustrated in FIGS. 5 to 8, retention channels 3a may extend into the well 2 through the peripheral wall surface 2a2 of the well 2. The retention channels 3a may be used for intermittent retention manipulation in which the target cell 4 is intermittently retained at tip ends of the retention channels 3a using the fluid draining from the well 2. The intermittent retention manipulation may cause the target cell 4 to rotate by the action of gravity or buoyancy to manipulate the posture of the target cell 4.

As in the third embodiment, one of the retention channels 3a may correspond to a first retention channel 3a used for first posture manipulation in which the posture of the target cell 4 is manipulated by rotating the target cell 4 about a first axis O1. The other retention channel 3a may correspond to a second retention channel 3a. The second retention channel 3a may extend into the well 2 through the peripheral wall surface 2a2 of the well 2. By intermittent retention manipulation in which the target cell 4 is intermittently retained at the tip end of the second retention channel 3a using the fluid draining from the well 2, the second retention channel 3a may be used for second posture manipulation in which the posture of the target cell 4 is manipulated by rotating the target cell 4 about a second axis O2 different from the first axis O1 by the action of gravity or buoyancy.

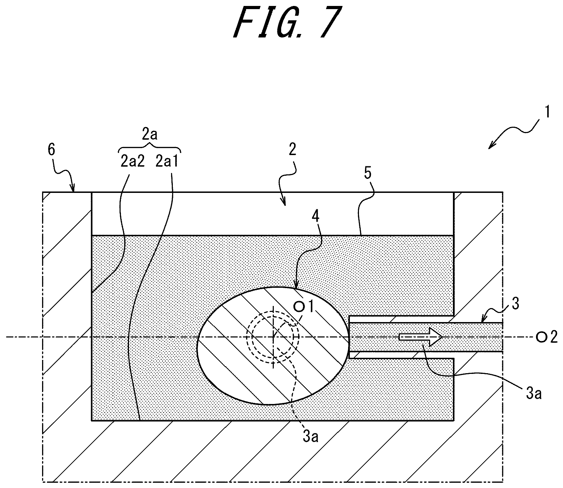

For example, as illustrated in FIGS. 5 to 7, by intermittent retention manipulation, the first retention channel 3a can cause the target cell 4 to rotate about the first axis O1 by the action of gravity. Specifically, after retention manipulation by draining the fluid as illustrated in FIG. 5, stopping the drainage of the fluid causes the target cell 4 to rotate by the action of gravity as illustrated in FIG. 6. Then, by restarting the drainage of the fluid as illustrated in FIG. 7, the target cell 4 in a rotated state can be retained again. When the density of the target cell 4 is greater than the density of the liquid 5, rotation occurs by the action of gravity. When the density of the target cell 4 is less than the density of the liquid 5, rotation occurs by the action of buoyancy. The second retention channel 3a can also cause the target cell 4 to rotate about the second axis O2 in the same manner.

In any of the embodiments described above, part or all of the fluid that has been drained from the well 2 may be returned to the well 2 manually using an appropriate instrument such as a pipette, or automatically using a pump or the like, while the target cell 4 is being retained by the retention channel 3a and treated, or after treatment is performed. Alternatively, to replenish the fluid that has been drained from the well 2, the fluid may be newly supplied to the well 2.

Although the embodiments of the present disclosure have been described above, the present disclosure is not limited to the aforementioned embodiments. The aforementioned embodiments can be modified in various manners without departing from the gist of the present disclosure.

## Claims

1. A cell housing device comprising:
a well; and
a posture manipulation device,
wherein
the well has an inner wall surface configured to accommodate a target cell immersed in a liquid,
the target cell is a cell or a cell aggregate,
the posture manipulation device has a retention channel,
the retention channel is configured to drain a fluid from the well via the inner wall surface of the well, to bring the target cell into a retained state in which the target cell is rotatably retained at a tip end of the retention channel, and
the posture manipulation device is configured to manipulate a posture of the target cell by rotating the target cell in the retained state.

2. The cell housing device according to claim 1, wherein
the posture manipulation device has a manipulation channel, and
the manipulation channel is configured to allow a fluid to flow via the inner wall surface of the well, to cause the target cell in the retained state to rotate in order to manipulate the posture of the target cell.

3. The cell housing device according to claim 2, wherein the retention channel and the manipulation channel extend in parallel with each other.

4. The cell housing device according to claim 2, wherein the retention channel and the manipulation channel extend at an angle to each other.

5. The cell housing device according to claim 4, wherein a tip end of the manipulation channel merges with the tip end of the retention channel.

6. The cell housing device according to claim 2, wherein the retention channel and the manipulation channel are provided separately.

7. The cell housing device according to claim 2, wherein each of the retention channel and the manipulation channel is configured to allow a fluid to flow via a bottom wall surface of the well.

8. The cell housing device according to claim 2, wherein
the manipulation channel corresponds to a first manipulation channel used for first posture manipulation in which the posture of the target cell is manipulated by rotating the target cell about a first axis,
the posture manipulation device has a second manipulation channel, and
the second manipulation channel configured to allow a fluid to flow via the inner wall surface of the well is used for second posture manipulation in which the posture of the target cell is manipulated by rotating the target cell in the retained state about a second axis different from the first axis.

9. The cell housing device according to claim 1, wherein
the retention channel extends into the well via a peripheral wall surface of the well, and
the retention channel is used for intermittent retention manipulation in which the target cell is intermittently retained at the tip end of the retention channel using a fluid draining from the well, and the intermittent retention manipulation causes the target cell to rotate by action of gravity or buoyancy to manipulate the posture of the target cell.

10. The cell housing device according to claim 9, wherein
the retention channel corresponds to a first retention channel used for first posture manipulation in which the posture of the target cell is manipulated by rotating the target cell about a first axis,
the posture manipulation device has a second retention channel,
the second retention channel extends into the well via the peripheral wall surface of the well, and
the second retention channel is used for intermittent retention manipulation in which the target cell is intermittently retained at a tip end of the second retention channel using a fluid draining from the well, and the intermittent retention manipulation causes second posture manipulation to be performed in which the posture of the target cell is manipulated by rotating the target cell about a second axis different from the first axis by action of gravity or buoyancy.
